# EUROPEAN PATENT APPLICATION

(11) **EP 2 455 750 A1**
(43) Date of publication of application: **23.05.2012**
(21) Application number: 10799801.5
(22) Date of filing: 12.07.2010
(51) Int. Cl.: G01N 27/62, G01N 33/68, C07K 14/595, C07K 14/605, C07K 14/695, C07K 14/805

(54) **METHOD FOR SPECIFIC CLEAVAGE OF N-C BOND IN PEPTIDE MAIN CHAIN**

(30) Priority: 16.07.2009 JP 2009167550
(71) Applicant: Public University Corporation Yokohama City University, Yokohama-shi, Kanagawa 236-0027 (JP)
(72) Inventor: TAKAYAMA, Mitsuo, Yokohama-shi Kanagawa 236-0027 (JP); SAKAKURA, Motoshi, Yokohama-shi Kanagawa 236-0027 (JP)
(74) Representative: Duckworth, Timothy John
(86) International application number: PCT/JP2010/061744
(87) International publication number: WO 2011/007743

(57) **Abstract**

The present invention provides a peptide degradation reagent with the following characteristics: 1) it has no marked toxicity such as carcinogenicity, 2) it does not produce metastable peaks resulting from excessive degradation property, 3) it does not produce multiply-charged ion peaks which are interference peaks, and 4) it can secure separation and sharpness of peaks. The present invention also provides a method for specifically cleaving N-Cα bonds on a peptide backbone using the above-described reagent, and a method of determining the amino acid sequence of a peptide utilizing this specific cleavage.

A method for specifically cleaving N-Cα bonds on the backbone of a peptide, comprising irradiating the peptide with laser light in the presence of 5-amino salicylic acid. A method for determining the amino acid sequence of a peptide, comprising irradiating the peptide with laser light in the presence of 5-amino salicylic acid to thereby specifically cleave N-Cα bonds on the peptide backbone. A reagent for specifically cleaving N-Cα bonds on a peptide backbone; a hydrogen radical-releasing reagent; a matrix reagent for MALDI-ISD; a matrix for MALDI-ISD; a peptide ionization reagent for MALDI-ISD; and a kit for MALDI-ISD.

## Description

### TECHNICAL FIELD

The present invention relates to a method for specifically cleaving N-Cα bonds in a peptide backbone. More specifically, the present invention relates to a method for specifically cleaving N-Cα bonds on a peptide backbone using 5-amino salicylic acid and laser light.

### BACKGROUND ART

The peptide backbone of proteins and polypeptides is a polymer consisting of units of -CaH-CO-NH-CαH- in which amino acid side chains R groups are attached to the carbon at the position α (-Cα-). The numbering of amino acids starts from the amino (N)-terminus and ends at the carboxy (C)-terminus. A protein or peptide consisting of a specific number (n) of amino acid residues may be expressed as NH₂-R₁-R₂-R₃-----Rₙ-₁-Rₙ-COOH in the one letter notation. The most useful information for protein identification is the amino acid sequence. When information on about 10 residues in a partial sequence located on the N-terminal side, C-terminal side or in the middle of a protein of interest is available, the protein can be easily identified using databases. Various methods for analyzing amino acid sequences have been developed and mass spectrometry (MS) is one of them. In mass spectrometry, samples are ionized under vacuum conditions; the resultant ions are separated according to their mass-to-charge number ratio (m/z); and the relative abundance of each ion is measured. Various ionization techniques such as electron ionization (ET), chemical ionization (CI), field desorption (FD), fast atom bombardment (FAB), matrix-associated laser desorption/ionization (MALDI) and electrospray ionization (ESI) have been developed. In order to separate ionized samples, various types of m/z analyzers such as magnetic sector type, quadrupole type, ion trap type, time-of-flight (TOF) type and Fourier transform ion cyclotron resonance type are used. The combination of MALDI and TOF is widely used in analyzing macromolecules such as polymers and proteins since this is a combination of an ion source applicable up to high molecular weight and an m/z analyzer applicable up to high molecular weight.

Direct sequencing of proteins using matrix-assisted laser desorption/ionization time of flight mass spectrometry (MALDI-TOF MS) was first reported by R S. Brown and J. J. Lennon (Non-Patent Document No. 1). Subsequently, this technique was applied to direct sequencing of various proteins (Non-Patent Documents Nos. 2 to 6); however, the mechanism by which specific cleavage reactions that enable amino acid sequencing of proteins occur through laser irradiation was totally unknown. Independently of the first report of Brown et al., the present inventors have also encountered with this phenomenon and demonstrated that this phenomenon occurs independently of ionization (Non-Patent Document No. 7). That is, the protonated molecule [M+H]⁺ produced upon acceptance of protons by peptides or proteins under MALDI conditions does not undergo the cleavage, but the neutral molecule M is cleaved through laser irradiation. Since the cleavage occurs inside the ion source, this phenomenon is called in-source decay (ISD).

Ionization in MALDI occurs by the ion-molecule reaction caused by explosive vaporization of a large excess of matrix that has absorbed photons when laser light is irradiated on a crystal system produced by mixing the matrix and a sample. A matrix in MALDI is an ionizing reagent which can serve both as a proton source and a proton receptor. It has been reported that 2,5-dihydroxybenzoic acid (2,5-DHB) is suitable as a matrix for specifically cleaving N-Cα bonds in peptides and proteins (Non-Patent Document No. 8). Subsequently, an excellent reagent 1,5-diaminonaphthalene (1,5-DAN) was reported by a Shimadzu Corp. group lead by Mr. Koichi Tanaka (Non-Patent Document No. 9) and confirmed by an overseas group (Non-Patent Document No. 10). However, 2,5-DHB has a "hot" property of promoting excessive degradation, and 1,5-DAN has sublimation property and carcinogenicity. Under these circumstances, other matrix chemicals superior to them have been demanded.

### DISCLOSURE OF THE INVENTION

### PROBLEM TO BE SOLVED BY THE INVENTION

It is an object of the present invention to provide a degradation reagent (matrix) having the following characteristics:
- a reagent which has no significant toxicity such as carcinogenicity.
- a reagent which does not produce metastable peaks resulting from excessive degradation property.
- a reagent which does not produce multiply-charged ion peaks that are interference peaks.
- a reagent which can secure separation and sharpness of peaks.

It is another object of the present invention to provide a method for specifically cleaving N-Cα bonds on a peptide backbone using the above-described degradation reagent; and a method for determining the amino acid sequence of a peptide utilizing the specific cleavage.

### MEANS TO SOLVE THE PROBLEM

When the present inventors irradiated peptides with laser light in the presence of 5-amino salicylic acid (5-ASA), N-Cα bonds alone of the peptide backbone were cleaved specifically. Analysis of the reaction products by MALDI-TOF MS allowed determination of their amino acid sequences. The present invention has been achieved based on these findings.

The present invention may be summarized as follows.
(1) A method for specifically cleaving N-Cα bonds on the backbone of a peptide, comprising irradiating the peptide with laser light in the presence of 5-amino salicylic acid.
(2) A method for determining the amino acid sequence of a peptide, comprising irradiating the peptide with laser light in the presence of 5-amino salicylic acid to thereby specifically cleave N-Cα bonds on the peptide backbone.
(3) The method according to (2) above, wherein 5-amino salicylic acid is used as a matrix for matrix-assisted laser desorption/ionization mass spectrometry.
(4) A reagent for specifically cleaving N-Cα bonds on a peptide backbone, comprising 5-amino salicylic acid.
(5) A hydrogen radical-releasing reagent comprising 5-amino salicylic acid.
(6) A matrix reagent for matrix-assisted laser desorption/ionization mass spectrometry, comprising 5-amino salicylic acid.
(7) A matrix for matrix-assisted laser desorption/ionization mass spectrometry, comprising 5-amino salicylic acid.
(8) A peptide ionization reagent for matrix-assisted laser desorption/ionization mass spectrometry, comprising 5-amino salicylic acid.
(9) A kit for matrix-assisted laser desorption/ionization mass spectrometry, comprising 5-amino salicylic acid.

### EFFECT OF THE INVENTION

5-ASA has both peptide ionization ability and hydrogen radical releasing ability in matrix-assisted laser desorption/ionization (MALDI) mass spectrometry, and does not show any particular toxicity. Since 5-ASA is capable of suppressing the internal energy of the produced ions at a low level, it does not produce either metastable peaks or multiply-charged ion peaks. Further, 5-ASA allows highly precise reading of information because the sharpness of peaks that is important for amino acid sequence analysis is secured.

The present specification encompasses the contents disclosed in the specification and/or drawings of Japanese Patent Application No. 2009-167550 based on which the present application claims priority.

### BRIEF DESCRIPTION OF THE DRAWINGS

Fig. 1 shows the principle of specific cleavage at N-Cα bonds by MALDI-TOF MS.
Fig. 2 shows c- and z-series ions generated by specific cleavage at N-Cα bonds in a peptide backbone.
Fig. 3 shows amino acid identification from an ISD spectrum.
Fig. 4 shows a MALDI-ISD spectrum of a peptide obtained with 5-ASA matrix.
Fig. 5 shows a MALDI-ISD spectrum of a phosphorylated peptide obtained with 5-ASA matrix.
Fig. 6 shows a MALDI-ISD spectrum of a di-phosphorylated peptide obtained with 5-ASA matrix.
Fig. 7 shows comparison of contaminant peaks in MALDI-ISD spectra of a peptide obtained with 1,5-DAN (1,5-diaminonaphthalene), 2,5-DHB (2,5-dihyrdoxybenzoic acid) and 5-ASA matrix.
Fig. 8 shows comparison of peak separation abilities in MALDI-ISD spectra of a peptide obtained with 1,5-DAN, 2,5-DHB and 5-ASA matrix.
Fig. 9 shows comparison of MALDI-ISD spectra of a sulfonated peptide (CCK33) obtained with 2,5-DHB and 5-ASA matrices.
Fig. 10 shows comparison of partial MALDI-ISD spectra (m/z 600-3700) of a sulfonated peptide (CCK33) obtained with 2,5-DHB and 5-ASA matrices.
Fig. 11 shows comparison of MALDI-ISD spectra of a protein (myoglobin) obtained with 2,5-DHB and 5-ASA matrices and measured in linear mode.
Fig. 12 shows comparison of partial MALDI-ISD spectra (m/z 4300-7500) of a protein (myoglobin) obtained with 2,5-DHB and 5-ASA matrices and measured in linear mode.
Fig. 13 shows comparison of MALDI-ISD spectra of a protein (myoglobin) obtained with 2,5-DHB and 5-ASA matrices and measured in reflectron mode.
Fig. 14 shows comparison of partial MALDI-ISD spectra (m/z 1500-5600) of a protein (myoglobin) obtained with 2,5-DHB and 5-ASA matrices and measured in reflectron mode.
Fig. 15 shows comparison of MALDI-ISD spectra of a peptide (glucagon) obtained with 2,5-DHB and 5-ASA matrices.
Fig. 16 shows comparison of partial MALDI-ISD spectra (m/z 650-3300) of a peptide (glucagon) obtained with 2,5-DHB and 5-ASA matrices.

### BEST MODE FOR CARRYING OUT THE INVENTION

Hereinbelow, embodiments of the present invention will be described in detail.

The present invention provides a method for specifically cleaving N-Cα bonds on a peptide backbone, comprising irradiating the peptide with laser light in the presence of 5-amino salicylic acid (5-ASA).

Generally, a peptide consists of two or more amino acids joined together by peptide bonds. A protein is a polypeptide in which about 20 species of L-α-amino acids are joined together by peptide bonds. The number of amino acids that constitute a peptide is not particularly limited. The number may be 2 to 200, preferably 10 to 50, and more preferably 15 to 25. When the number of amino acids that constitute a peptide is greater than 100, the peptide may be used in the method of N-Cα bond specific cleavage according to the present invention after it is digested with an enzyme such as trypsin and separated/fractionated by liquid chromatography. The species of amino acids which constitute a peptide is not particularly limited. They may be any amino acids. Further, the amino acids may be modified amino acids, e.g., acetylated, methylated, phosphorylated, glycosylated or sulfonated. The term "peptide" includes proteins, polypeptides other than proteins, oligopeptides, glycopeptides, lipoproteins or the like.

5-ASA is a known compound and commercially available. 5-ASA is used as an active ingredient in mesalazine formulations which are therapeutics for ulcerative colitis and Crohn's disease.

In the method of specific cleavage at N-Cα bonds on a peptide backbone according to the present invention, 5-ASA may be used in an amount of 5000 to 50000 mols, preferably 5000 to 10000 mols, per 1 mol of the peptide.

As the laser light, an ultraviolet laser such as nitrogen laser of wavelength 337 nm or Nd:YAG laser of wavelength 226 nm may be used. Of these, nitrogen laser of wavelength 337 nm is preferable. The accelerating voltage for generated ions may be about 20 to 25 kV but is not limited to this range.

It is possible to specifically cleave at N-Cα bonds on a peptide backbone by irradiating the peptide with laser light in the presence of 5-ASA. Therefore, the present invention encompasses a reagent for specifically cleaving N-Cα bonds on a peptide backbone, comprising 5-ASA.

It is possible to determine the amino acid sequence of a peptide by applying the degradation products of the peptide produced by the method of specific cleavage at N-Cα bonds on a peptide backbone according to the present invention to a mass spectrometer to thereby obtain mass spectra. The present invention also encompasses a method of determining the amino acid sequence of a peptide, comprising irradiating the peptide with laser light in the presence of 5-amino salicylic acid to thereby specifically cleave at N-Cα bonds on the peptide backbone.

5-ASA may be used as a matrix in mass spectrometry, preferably in matrix-assisted laser desorption/ionization mass spectrometry (MALDI MS), more preferably in MALDI-ISD. The present invention also encompasses a matrix reagent for MALDI, comprising 5-ASA.

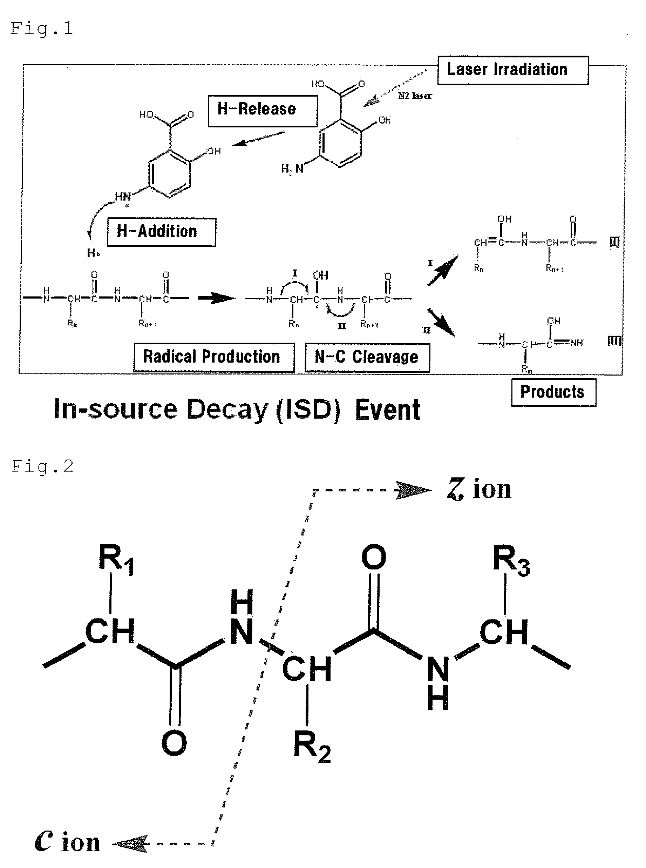

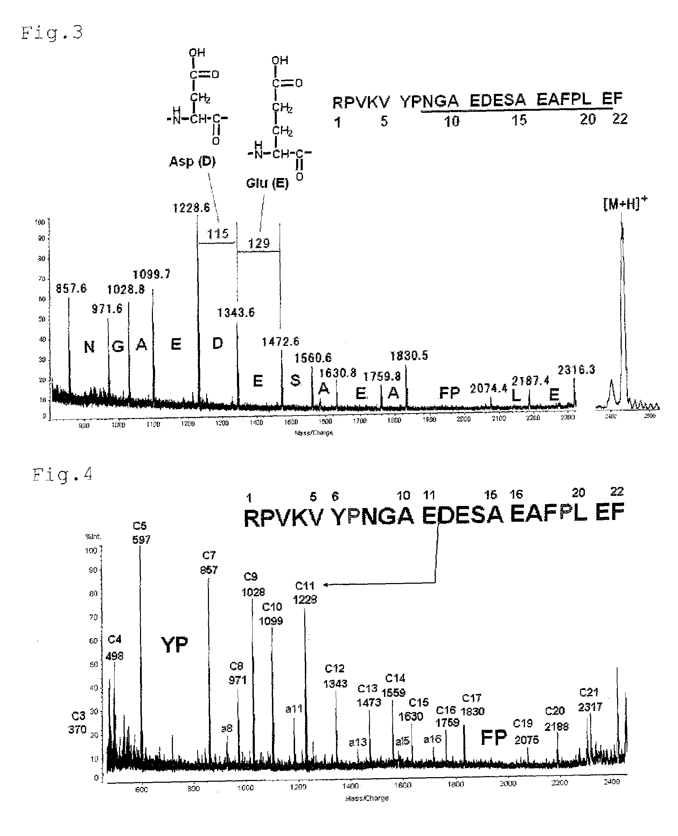

When 5-ASA is used as a matrix in MALDI, a mixture (mixed crystal) of a sample (peptide) and a matrix (5-ASA) is irradiated with laser light. The peptide is present on the surfaces of 5-ASA crystals, with the carbonyl oxygen on the peptide backbone having been bound to the hydroxyl group of 5-ASA by hydrogen bond in advance. Upon absorption of laser photons, 5-ASA is excited to dissociate into hydrogen radicals and 5-ASA radicals. The hydrogen radical continues to bind to the carbonyl oxygen on the peptide backbone to yield a hydroxyl group, whereas the carbonyl carbon becomes a radical. Subsequently, the NH-Cα bond which is at the α position relative to the radical site undergoes simple cleavage (α cleavage) (Fig. 1). Ions generated at this time are c- and z-ions (Fig. 2). Thus, degradation of the peptide (i.e., fragmentation) occurring in the ion source simultaneously with or immediately after ionization is called in-source fragmentation or in-source decay (ISD). Peptide fragment ions produced by MALDI-ISD consist mainly of ladder-like c-ion peak groups in which N-terminus is preserved predominantly. In these peak groups, mass difference Δ (m/z) between neighboring peaks means the mass of an amino acid residue. Therefore, when mass differences in a ladder-like peak group are arranged in order, a partial amino acid sequence can be obtained (Fig. 3).

When used as a matrix in MALDI, 5-ASA was superior in the ability to separate individual peaks as compared to conventional MALDI-ISD data.

5-ASA has both MALDI matrix function (peptide ionizing ability) and hydrogen radical releasing ability. Furthermore, 5-ASA has a high ability of hydrogen radical transfer reaction (ISD). Therefore, the present invention encompasses a hydrogen radical-releasing reagent comprising 5-ASA. Also, the present invention encompasses a peptide ionization reagent for MALDI, comprising 5-ASA.

When used as a matrix in MALDI, 5-ASA is dissolved in 0.05-0.5% (preferably 0.3%) TFA-containing aqueous acetonitrile solution (40-70% (preferably 50%), v/v) and the resultant saturated solution may be used as a matrix solution. Although 5-ASA is sparingly soluble in hydrophilic solvents, its solubility can be increased markedly by addition of 0.3%-0.5%TFA.

The 5-ASA concentration in the matrix solution may be 1-20 pmol/µl, preferably 5-10 pmol/µl. Buffer components, picolinic acid, and the like may be added to the matrix solution. The matrix solution may be mixed with a peptide sample so that the molar ratio of 5-ASA to the peptide falls in the range of about 5000-50000, preferably about 5000-10000. The mixed solution of matrix solution and peptide sample may be applied dropwise to a plate and air-dried to remove the solvent. When 5-ASA is used, needle crystals grow and peptide molecules begin to cover the surfaces of matrix crystals. Since amino groups of 5-ASA molecules are exposed on the surfaces of matrix crystals, the peptide molecules cover matrix crystals in such a manner that they mount on groups of the amino group. The present invention encompasses a matrix for MALDI, comprising 5-ASA. In addition to 5-ASA, the matrix may also contain buffer components, picolinic acid and the like.

The present invention is applicable to direct sequencing of peptides and proteins utilizing mass spectrometry, especially MALDI-TOF MS. Thus, the present invention is applicable to the fields of proteomics and protein chemistry.

5-ASA has both peptide ionization ability and hydrogen radical releasing ability in MALDI-TOF MS, and does not show any particular toxicity. Since 5-ASA is capable of suppressing the internal energy of the produced ions at a low level, it does not produce either metastable peaks or multiply-charged ion peaks. Further, 5-ASA allows highly precise reading of information because the sharpness of peaks that is important for amino acid sequence analysis is secured. Thus, 5-ASA resolves most of the problems with the conventional matrices and its superiority is clear.

Further, the present invention provides a kit for MALDI, comprising 5-ASA. This kit may further comprise other matrix reagents (such as α-cyano-4-hydroxycinnamic acid (CHCA), sinapinic acid, 2,5-dihydroxybenzoic acid (2,5-DHB), 3-hydroxypicolinic acid and ferulic acid), buffer components, picolinic acid, handling instructions, cautions, written explanation of the contents, etc., standard peptides, and so on.

### EXAMPLES

Hereinbelow, the present invention will be described more specifically with reference to the following Examples. However, the present invention is not limited to these Examples.

### [Example 1]

### Experimental Methods:

As a mass spectrometer, a time-of-flight (TOF) mass spectrometer AXIMA-CFR (Shimadzu Corp., Kyoto) equipped with matrix-assisted laser desorption/ionization (MALDI) processes was used. As laser light, a nitrogen laser of wavelength 337 nm was used. The acceleration voltage for generated ions was 20 kV Used as sample peptides were adrenocorticotropic hormone (ACTH) fragment ACTH18-39 (Mr 2465.7) and ACTH18-35 (Mr 1979.1), mono-phosphorylated ACTH18-35 (Mr 2059.1) and di-phosphorylated ACTH18-35 (Mr 2139.1). These peptides were purchased from Peptide Institute (Minoh, Osaka) and used without processing. As matrices, 2,5DHB and 1,5-DAN were purchased from Sigma Aldrich (Steinheim, Germany), and 5-ASA from Tokyo Chemical Industry (Tokyo). These chemicals were used without recrystallization. Aqueous solution of the sample peptide (5 µl) was mixed with 5 µl of matrix solution (each matrix saturated in 50% aqueous acetonitrile solution). One µl of the resultant sample solution was coated on the sample target and air-dried to thereby prepare sample crystals. The target coated with the sample crystals was applied to the mass spectrometer.

### Results and Discussion:

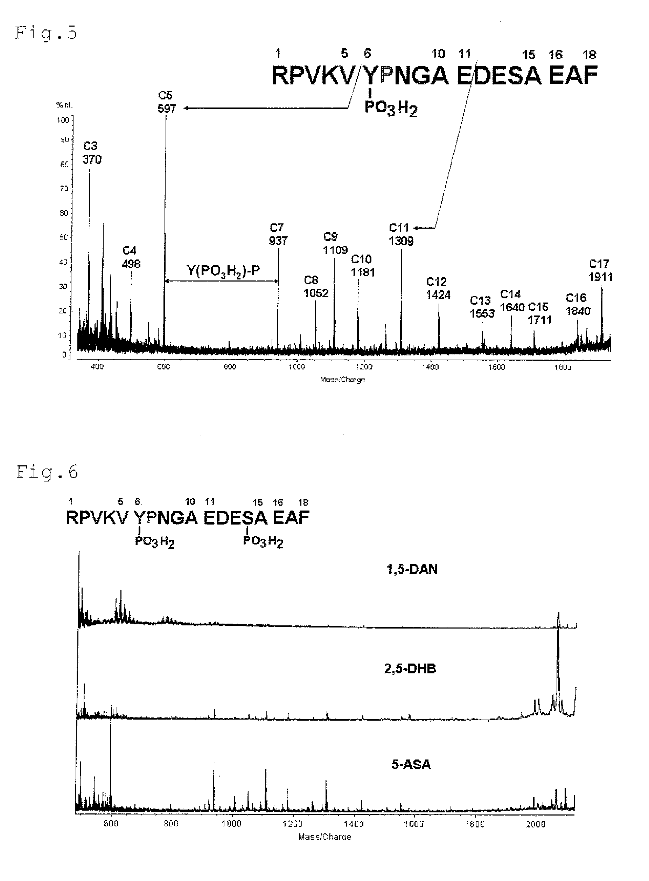

The MALDI-ISD spectrum of peptide ACTH18-39 obtained with 5-ASA as a matrix is shown in Fig. 4. Not only protonated molecules [M+H]⁺ but also N-terminal c-ions from c3 to c21 were observed in the spectrum, which made it possible to read the amino acid sequence of the peptide. This shows that 5-ASA is not only useful as an ionization matrix for NIALDI but also has ISD function. However, since complete cleavage does not occur on the N-terminal side of proline residue, c6 and c18 were not observed. In order to examine whether this matrix is also effective for a post-translationally modified peptide or not, MALDI-ISD spectrum of mono-phosphorylated ACTH18-35 having the tyrosine at position 6 phosphorylated was obtained (Fig. 5). In the resultant spectrum, c-ions from c3 to c17 were observed (with the exception of c6 ion corresponding to proline residue cleaved on the N-terminal side), and it was possible to read the amino acid sequence. Further, since no degradation or loss of phosphate group occurred, it was possible to confirm the presence and specify the site of phosphate group from the mass difference between c6 and c8.

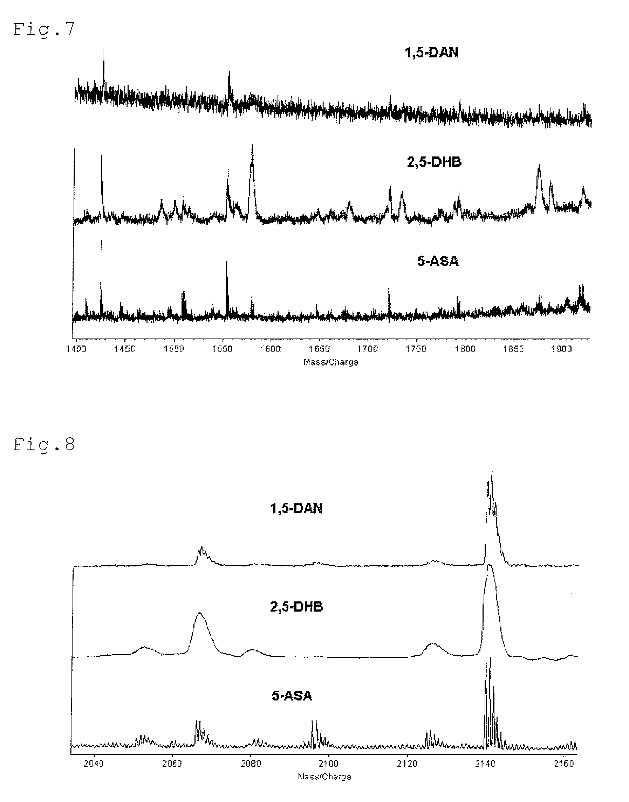

Di-phosphorylated ACTH18-35 (a di-phosphorylated peptide) was measured with the three matrices 1,5-DAN, 2,5-DHB and 5-ASA, followed by comparison of the resultant MALDI-ISD spectra (Fig. 6). With 1,5-DAN, it was possible to read the amino acid sequence from c7 to c17 but signal intensities were weak. Further, since c4 and c5 were not observed, it was impossible to confirm the phosphorylation of tyrosine at position 6. This is because when 1.5-DAN is used, peaks of matrix clusters appear below m/z 800 and interfere with the appearance of signal peaks. With 2,5-DHB, sequence information from c5 to c17 was obtained, but analysis was difficult to perform because signal intensities were weak and peak separation was ambiguous. On the other hand, with 5-ASA, fragment ions were clearly observed from c3 to c17, and yet no desorption of phosphate groups occurred. Therefore, it was possible to confirm the presence and specify the site of phosphate groups from mass differences between peaks. Further, as shown in Fig. 7, broad metastable peaks were observed and interfered with analysis, particularly in the case where 2,5-DHB was used. This shows that, with 2,5-DHB, the internal energy of generated ions is abundant, causing excessive degradation. In connection with this, 5-ASA is marked high the ability to separate isotope peaks neighboring protonated molecule [M+H]⁺ peaks, which allows high accuracy in analysis (see Fig. 8).

### [Example 2]

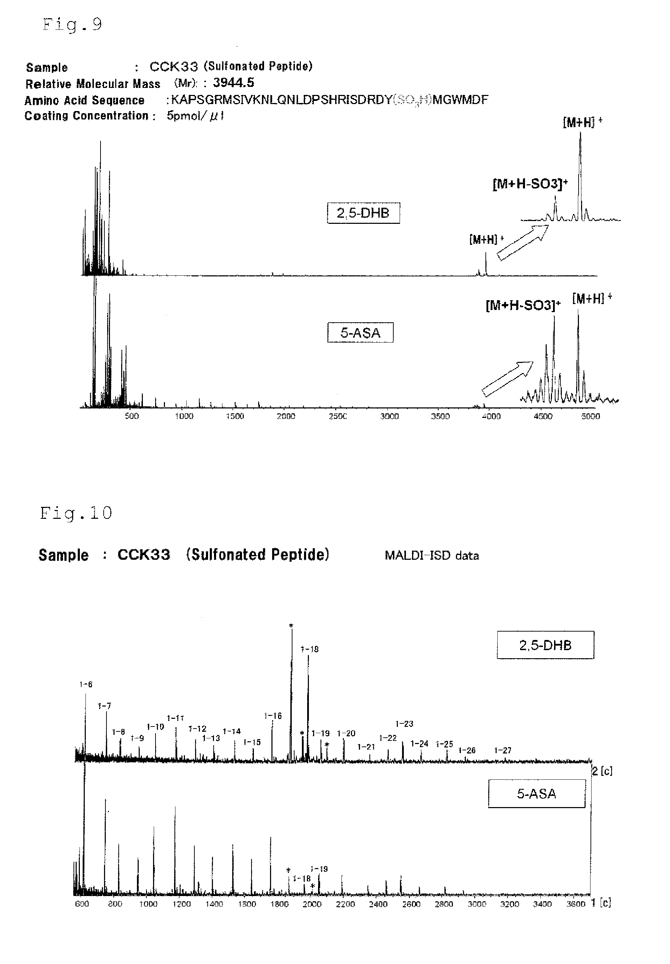

Experiments were performed in the same manner as in Example 1, except that CCK33 (a sulfonated peptide) (Peptide Institute; Minoh, Osaka) was used as a sample peptide and that individual matrices saturated in 50% acetonitrile aqueous solution containing 0.3% TFA were used as matrix solutions. The results are shown in Figs. 9 and 10.

### Results and Discussion:

Fig. 9 shows comparison of MALDI-ISD spectra of sulfonated peptide CCK33 obtained with 2,5-DHB and 5-ASA matrices. Fig. 10 shows the same spectra, with the horizontal axes expanded. In these spectra, not only protonated molecules [M+H]⁺ but also N-terminal c-ions from c6 to c27 (with the exception of c17 corresponding to proline residue cleaved on the N-terminal side) were observed; thus, it was possible to read the amino acid sequence. In particular, the spectrum obtained with 5-ASA showed lower intensity of metastable peaks (interfering peaks; indicated with asterisk "*") as compared to those observed in the spectrum obtained with 2,5-DHB. This means that 5-ASA is superior to 2,5-DHB in that it yields less interfering peaks. Although desorption of sulfonic acid was observed with both matrices (Fig. 9), the sulfonic acid bound to tyrosine at position 27 (Tyr27) was observed as it remained bound to c27-ion. Thus, it was possible to determine the position of the sulfonic acid.

### [Example 3]

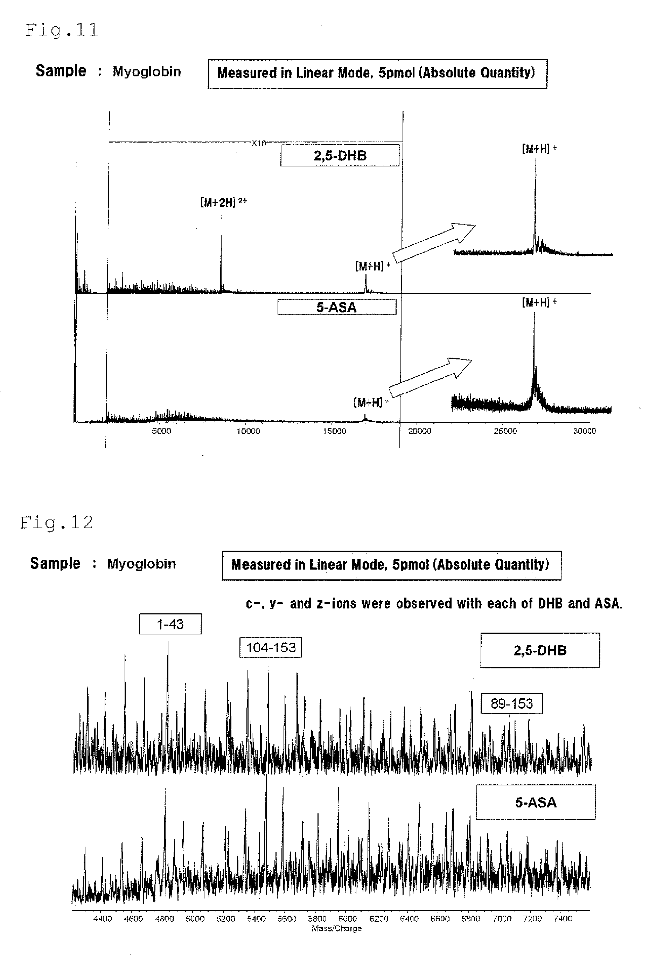

Experiments were performed in the same manner as in Example 1, except that myoglobin (equine myocardial protein; Mr 16951.4; Sigma Aldrich, Steinheim, Germany) (PDB data: http://wwwpdb.org/pdb/explore/explore,do?structureId=IWLA) was used as a sample peptide and that individual matrices saturated in 50% acetonitrile aqueous solution containing 0.3% TFA were used as matrix solutions. The results are shown in Figs. 11, 12, 13 and 14.

### Results and Discussion:

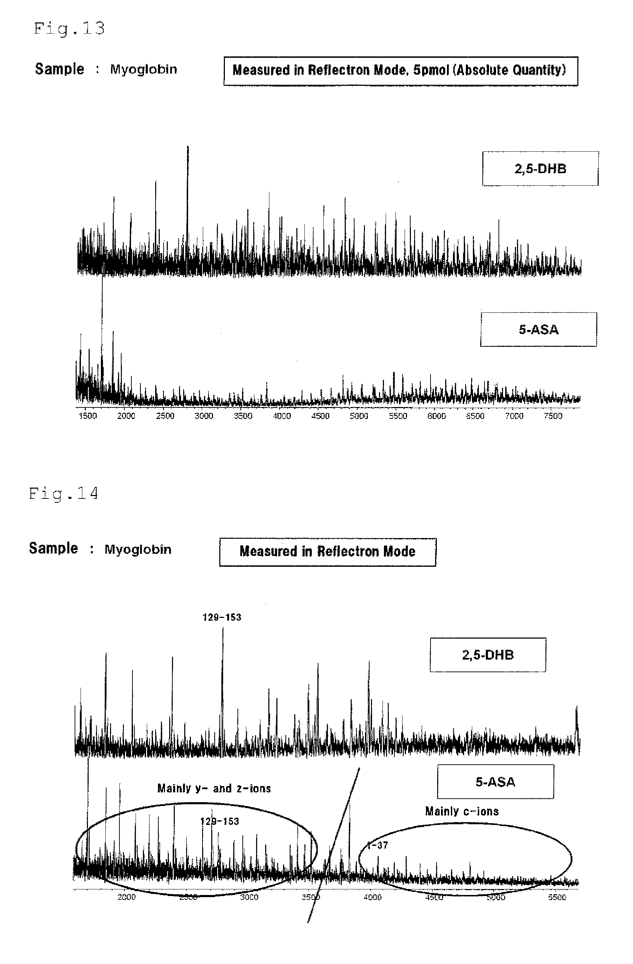

Fig. 10 shows comparison of MALDI-ISD spectra of a protein myoglobin obtained with matrices 2,5-DHB and 5-ASA using the spectrometer in the linear mode. Fig. 11 shows partial spectra of the same, with the horizontal axes expanded. Fig. 13 shows comparison of MALDI-ISD spectra of a protein myoglobin obtained with matrices 2,5-DHB and 5-ASA using the spectrometer in the reflectron mode. Fig. 14 shows partial spectra of the same, with the horizontal axes expanded. In the above-described spectra, not only protonated molecules [M+H]⁺ but also N-terminal c-ions from c39 to c65 and C-terminal y-ions and (z+2)-ions were observed; thus, it was possible to read the amino acid sequence. These results demonstrate that 5-ASA is applicable to analysis of not only peptides but also proteins.

### [Example 4]

Experiments were performed in the same manner as in Example 1, except that glucagon (hormonal peptide; Mr 3480.5; Peptide Institute, Minoh, Osaka) (PDB data: http://www.pdb.org/pdb/explore/explore.do?structureId=IGCN) was used as a sample peptide and that individual matrices saturated in 50% acetonitrile aqueous solution containing 0.3% TFA were used as matrix solutions.

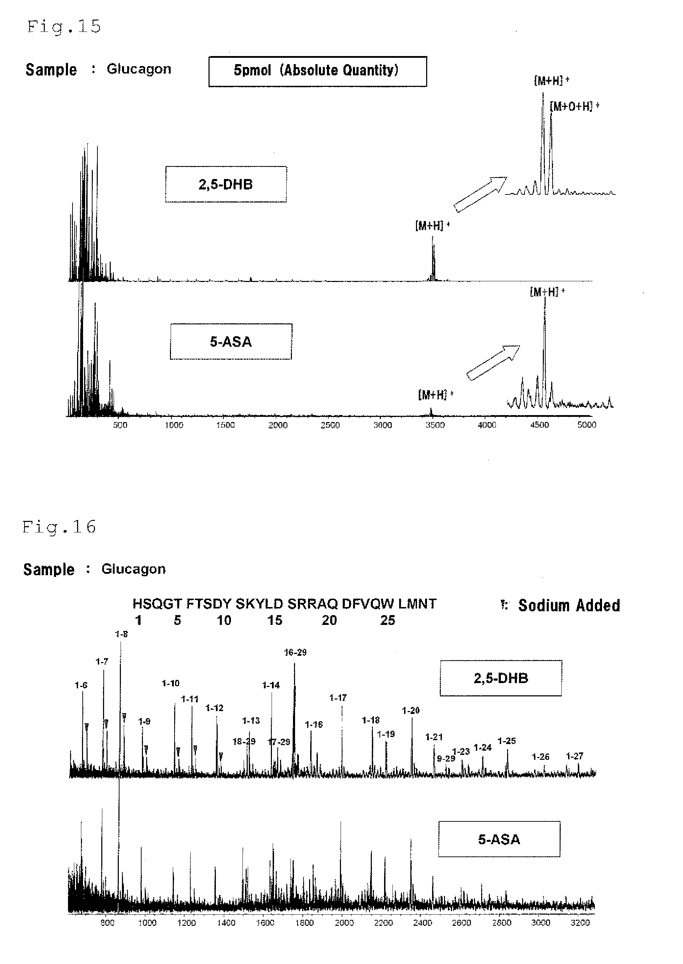

The results are shown in Figs. 15 and 16.

### Results and Discussion:

Fig.15 shows comparison af MALDI-ISD spectra of glucagon (a hormonal peptide with a helix structure) that was obtained with matrices 2,5-DHB and 5-ASA. Fig. 16 shows partial spectra of the same, with the horizontal axes expanded. In these spectra, not only protonated molecules [M+H]⁺ but also N-terminal c-ions from c6 to c27 were observed; thus, it was possible to read the amino acid sequence. With 2,5-DHB, a peak of oxidized molecule [M+O+H]⁺ was observed (Fig. 15); and peaks of sodium ion-added molecules were also observed (Fig. 16). On the other hand, with 5-ASA, neither oxidized molecules nor sodium ion-added molecules were observed, showing that 5-ASA is superior in analytical property.

The characteristics and advantages of 5-ASA are summarized below.
1. It has no marked toxicity such as carcinogenicity.
2. It does not produce metastable peaks which interfere with analysis.
3. It does not produce multiply-charged ion peaks which interfere with analysis.
4. It is superior in peak separation property and provides high accuracy in the reading of information.

All publications, patents and patent applications cited herein are incorporated herein by reference in their entirety.

### INDUSTRIAL APPLICABILITY

The present invention is applicable to the fields of proteomics and protein chemistry

### SEQUENCE LISTING FREE TEXT

<SEQ ID NO: 1>
   SEQ ID NO: 1 shows the amino acid sequence of an adrenocorticotropic hormone (ACTH) fragment ACTH18-39.
   Source: Human
   Amino Acid Sequence: RPVKV YPNGA EDESA EAFPL EF
<SEQ ID NO: 2>
   SEQ ID NO: 2 shows the amino acid sequence of an adrenocorticotropic hormone (ACTH) fragment ACTH18-35.
   Source: Human
   Amino Acid Sequence: RPVKV YPNGA EDESAEAF
<SEQ ID NO: 3>
   SEQ ID NO: 3 shows the amino acid sequence of CCK33.
   Source: Human
   Amino Acid Sequence:
   KAPSGRMSIVKNLQNLDPSHRISDRDY(SO3H)MGWMDF-NH2
<SEQ ID NO: 4>
   SEQ ID NO: 4 shows the amino acid sequence of myoglobin (equine myocardial protein).
   Source: Equine
   Amino Acid Sequence: 
GLSDEGWQQVLNVWGKVEADIAGHGQEVLIRLFTGHPETLEKFDKFKHLKTEAE
MKASEDLKKHGTVVLTALGGILKKKGHHEAELKPLAQSHATKHKIPIKYLEFISDAI
IHVLHSKHPGDFGADAQGAMTKALELFRNDIAAKYKELG FQG
SEQ ID NO: 5 shows the amino acid sequence of glucagon (hormonal peptide). Source: Porcine
   Amino Acid Sequence: HSQGTFTSDYSKYLDSRRAQDFVQWLMNT

### PRIOR ART LITERATURE

### Non-Patent Documents

[Non-Patent Document No. 1]
   R. R. Brown, and J. J. Lennon: Anal. Chem. 67 (1995) 3990.
[Non-Patent Document No. 2]
   J. J. Lennon, and K. A. Walsh: Protein Science 6 (1997) 2446.
[Non-Patent Document No. 3]
   D. C. Reiber, T. A. Grover, and R. S. Brown: Anal. Chem. 70 (1998) 673.
[Non-Patent Document No. 4]
   V Katta, D. T. Chow, and M. F. Rohde: Anal. Chem. 70 (1998) 4410.
[Non-Patent Document No. 5]
   J. J. Lennon, and K.A. Walsh: Protein Science 8 (1999) 2487.
[Non-Patent Document No. 6]
   M. Takayama, and A. Tsugita: Electrophoresis 21 (2000) 1670.)8-12.
[Non-Patent Document No. 7]
   M. Takayama, and A. Tsugita: Int. J. Mass Spectrom. 181 (1998) L1.
[Non-Patent Document No. 8]
   M. Takayama: J.Am.Soc.Mass Spectrom., 12(2001)420.
[Non-Patent Document No. 9]
   M. Takayama: J.Am.Soc.Mass.Spectrom., 12(2001)1044.
[Non-Patent Document No. 10]
   Y Fukuyama et al: J.Mass Spectrom., 41(2006)191.
[Non-Patent Document No. 11]
   K. Demeure et al: Anal. Chem., 79(2007)8678.

## Claims

1. A method for specifically cleaving N-Cα bonds on the backbone of a peptide, comprising irradiating the peptide with laser light in the presence of 5-amino salicylic acid.

2. A method for determining the amino acid sequence of a peptide, comprising irradiating the peptide with laser light in the presence of 5-amino salicylic acid to thereby specifically cleave N-Cα bonds on the peptide backbone.

3. The method according to claim 2, wherein 5-amino salicylic acid is used as a matrix for matrix-assisted laser desorption/ionization mass spectrometry.

4. A reagent for specifically cleaving N-Cα bonds on a peptide backbone, comprising 5-amino salicylic acid.

5. A hydrogen radical-releasing reagent comprising 5-amino salicylic acid.

6. A matrix reagent for matrix-assisted laser desorption/ionization mass spectrometry, comprising 5-amino salicylic acid.

7. A matrix for matrix-assisted laser desorption/ionization mass spectrometry, comprising 5-amino salicylic acid.

8. A peptide ionization reagent for matrix-assisted laser desorption/ionization mass spectrometry, comprising 5-amino salicylic acid.

9. A kit for matrix-assisted laser desorption/ionization mass spectrometry, comprising 5-amino salicylic acid.


SEQUENCE LISTING

<110>  Public University Corporation Yokohama City University

<120>  A method for cleavage specific for peptide main chain N-Cα bond

<130>  FP-146PCT

<140>  JP P2009-167550
<141>  2009-07-16

<160>  5

<170>  PatentIn version 3.1

<210>  1
<211>  22
<212>  PRT
<213>  Homo sapiens

<400>  1

Arg Pro Val Lys Val Tyr Pro Asn Gly Ala Glu Asp Glu Ser Ala Glu
1               5                   10                  15

Ala Phe Pro Leu Glu Phe
            20

<210>  2
<211>  18
<212>  PRT
<213>  Homo sapiens

<400> 2

Arg Pro Val Lys Val Tyr Pro Asn Gly Ala Glu Asp Glu Ser Ala Glu
1               5                   10                  15

Ala Phe

<210> 3
<211> 33
<212> PRT
<213> Homo sapiens

<400> 3

Lys Ala Pro Ser Gly Arg Met Ser Ile Val Lys Asn Leu Gln Asn Leu
1               5                   10                  15

Asp Pro Ser His Arg Ile Ser Asp Arg Asp Tyr Met Gly Trp Met Asp
            20                  25                  30

Phe

<210> 4
<211> 153
<212> PRT
<213> Equus caballus

<400> 4

Gly Leu Ser Asp Glu Gly Trp Gln Gln Val Leu Asn Val Trp Gly Lys
1               5                   10                  15

Val Glu Ala Asp Ile Ala Gly His Gly Gln Glu Val Leu Ile Arg Leu
            20                  25                  30

Phe Thr Gly His Pro Glu Thr Leu Glu Lys Phe Asp Lys Phe Lys His
        35                  40                  45

Leu Lys Thr Glu Ala Glu Met Lys Ala Ser Glu Asp Leu Lys Lys His
    50                  55                  60

Gly Thr Val Val Leu Thr Ala Leu Gly Gly Ile Leu Lys Lys Lys Gly
65                  70                  75                  80

His His Glu Ala Glu Leu Lys Pro Leu Ala Gln Ser His Ala Thr Lys
                85                  90                  95

His Lys Ile Pro Ile Lys Tyr Leu Glu Phe Ile Ser Asp Ala Ile Ile
            100                 105                 110

His Val Leu His Ser Lys His Pro Gly Asp Phe Gly Ala Asp Ala Gln
        115                 120                 125

Gly Ala Met Thr Lys Ala Leu Glu Leu Phe Arg Asn Asp Ile Ala Ala
    130                 135                 140

Lys Tyr Lys Glu Leu Gly Phe Gln Gly
145                 150

<210>  5
<211>  29
<212>  PRT
<213>  Sus scrofa

<400>  5

His Ser Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr
            20                  25